(19) Europäisches Patentamt / European Patent Office / Office européen des brevets

(11) **EP 2 710 955 A1**

(12) **EUROPEAN PATENT APPLICATION**

(43) Date of publication:
26.03.2014 Bulletin 2014/13

(21) Application number: 13188254.0

(22) Date of filing: 20.07.2007

(51) Int Cl.:
*A61B 5/083* (2006.01)  *G01N 27/49* (2006.01)
*G01N 33/497* (2006.01)

(84) Designated Contracting States:
AT BE BG CH CY CZ DE DK EE ES FI FR GB GR HU IE IS IT LI LT LU LV MC MT NL PL PT RO SE SI SK TR

(30) Priority: 21.07.2006 GB 0614504
19.04.2007 GB 0707553

(62) Document number(s) of the earlier application(s) in accordance with Art. 76 EPC:
12188800.2 / 2 548 505
07789043.2 / 2 043 518

(71) Applicant: **Anaxsys Technology Limited
Send, Nr. Woking
Surrey GU23 7EF (GB)**

(72) Inventors:
• **Varney, Mark
  Bishopstoke, Hampshire SO50 8PU (GB)**
• **Garrett, Michael
  Woking, Surrey GU22 7XR (GB)**

(74) Representative: **Irvine, Jonquil Claire
Harrison Goddard Foote LLP
8th Floor
140 London Wall
London EC2Y 5DN (GB)**

Remarks:
•This application was filed on 11-10-2013 as a divisional application to the application mentioned under INID code 62.
•Claims filed after the date of filing of the application (Rule 68(4) EPC).

(54) **Gas sensor**

(57) The present invention provides a sensor (2) for sensing water vapour in exhaled breath, the sensor comprising: a sensing element (8, 40) disposed to be exposed to the exhaled breath, the sensing element comprising: a working electrode (12, 44); a counter electrode (14, 46); and a layer of ion exchange material (48) extending between the working electrode and the counter electrode; whereby, when in use, contact of the ion exchange layer with water vapour in exhaled breath forms an electrical contact between the working and counter electrodes and the output of the sensor can be used to generate a display representative of the change in concentration of water vapour over time in a breath exhalation.

Figure 2

## Description

The present invention is related to a sensor for detecting gaseous substances, in particular a sensor for detecting the presence of substances in a gaseous phase or gas stream. The sensor is particularly suitable for, but not limited to, the detection of carbon dioxide. The sensor finds particular use as a capnographic sensor for detecting and measuring the concentration of gases, such as carbon dioxide, in the exhaled breath of a person or animal. The sensor may also be used to determine the moisture content or humidity of a gas stream, for example a stream of exhaled breath.

The analysis of the carbon dioxide content of the exhaled breath of a person or animal is a valuable tool in assessing the health of the subject. In particular, measurement of the carbon dioxide concentration allows the extent and/or progress of various pulmonary and/or respiratory diseases to be estimated, in particular asthma and chronic obstructive lung disease (COPD).

Carbon dioxide can be detected using a variety of analytical techniques and instruments. The most practical and widely used analysers use spectroscopic infrared absorption as a method of detection, but the gas may also be detected using mass spectrometry, gas chromatography, thermal conductivity and others. Although most analytical instruments, techniques and sensors for carbon dioxide measurement are based on the physicochemical properties of the gas, new techniques are being developed which utilise electrochemistry, and an assortment of electrochemical methods have been proposed. However, it has not been possible to measure carbon dioxide ($CO_2$) gas directly using electrochemical techniques. Indirect methods have been devised, based on the dissolution of the gas into an electrolyte with a consequent change in the pH of the electrolyte. Other electrochemical methods use high temperature catalytic reduction of carbon dioxide. However, these methods are generally very expensive, cumbersome to employ and often display very low sensitivities and slow response times. These drawbacks render them inadequate for analyzing breath samples, in particular in the analysis of tidal breathing.

A more recently applied technique is to monitor a specific chemical reaction in an electrolyte that contains suitable organometallic ligands that chemically interact following the pH change induced by the dissolution of the carbon dioxide gas. The pH change then disturbs a series of reactions, and the carbon dioxide concentration in the atmosphere is then estimated indirectly according to the change in the acid-base chemistry.

Carbon dioxide is an acid gas, and interacts with water, and other (protic) solvents. For example, carbon dioxide dissolves in an aqueous solution according to the following reactions:

$$CO_2 + H_2O \Leftrightarrow H_2CO_3 \qquad (1)$$

$$H_2CO_3 \Leftrightarrow HCO_3^- + H^+ \qquad (2)$$

$$HCO_3^- \Leftrightarrow CO_3^{2-} + H^+ \qquad (3)$$

It will be appreciated that, as more carbon dioxide dissolves, the concentration of hydrogen ions ($H^+$) increases.

The use of this technique for sensing carbon dioxide has the disadvantage that when used for gas analysis in the gaseous phase the liquid electrolyte must be bounded by a semi-permeable membrane. The membrane is impermeable to water but permeable to various gases, including carbon dioxide. The membrane must reduce the evaporation of the internal electrolyte without seriously impeding the permeation of the carbon dioxide gas. The result of this construction is an electrode which works well for a short period of time, but has a long response time and in which the electrolyte needs to be frequently renewed.

WO 04/001407 discloses a sensor comprising a liquid electrolyte retained by a permeable membrane, which overcomes some of these disadvantages. However, it would be very desirable to provide a sensor that does not rely on the presence and maintenance of a liquid electrolyte.

US 4,772,863 discloses a sensor for oxygen and carbon dioxide gases having a plurality of layers comprising an alumina substrate, a reference electrode source of anions, a lower electrical reference electrode of platinum coupled to the reference source of anions, a solid electrolyte containing tungsten and coupled to the lower reference electrode, a buffer layer for preventing the flow of platinum ions into the solid electrolyte and an upper electrode of catalytic platinum.

GB 2,287,543 A discloses a solid electrolyte carbon monoxide sensor having a first cavity formed in a substrate, communicating with a second cavity in which a carbon monoxide adsorbent is located. An electrode detects the partial pressure of oxygen in the carbon monoxide adsorbent. The sensor of GB 2,287,543 is very sensitive to the prevailing temperature and is only able to measure low concentrations of carbon monoxide at low temperatures with any sensitivity. High temperatures are necessary in order to measure carbon monoxide concentrations that are higher, if complete saturation of the sensor is to be avoided. This renders the sensor impractical for measuring gas compositions over a wide range of concentrations.

GB 2,316,178 A discloses a solid electrolyte gas sensor, in which a reference electrode is mounted within a cavity in the electrolyte. A gas sensitive electrode is provided on the outside of the solid electrolyte. The sensor is said to be useful in the detection of carbon dioxide and sulphur dioxide. However, operation of the sensor requires heating to a temperature of at least 200°C, more preferably from 300 to 400°C. This represents a major drawback in the practical applications of the sensor.

Sensors for use in monitoring gas compositions in heat treatment processes are disclosed in GB 2,184,549 A. However, as with the sensors of GB 2,316,178, operation at high temperatures (up to 600°C) is disclosed and appears to be required.

Accordingly, there is a need for a sensor that does not rely on the presence of an electrolyte in the liquid phase or high temperature catalytic method, that is of simple construction and may be readily applied to monitor gas compositions at ambient conditions.

EP 0 293 230 discloses a sensor for detecting acidic gases, for example carbon dioxide. The sensor comprises a sensing electrode and a counter electrode in a body of electrolyte. The electrolyte is a solid complex having ligands that may be displaced by the acidic gas. A similar sensor arrangement is disclosed in US 6,454,923.

A particularly effective sensor is disclosed in pending international application No. PCT/GB2005/003196. The sensor comprises a sensing element disposed to be exposed to the gas stream, the sensing element comprising a working electrode; a counter electrode; and a solid electrolyte precursor extending between and in contact with the working electrode and the counter electrode; whereby the gas stream may be caused to impinge upon the solid electrolyte precursor such that water vapour in the gas stream at least partially hydrates the precursor to form an electrolyte in electrical contact with the working electrode and the counter electrode.

It has been found that, while the sensor of PCT/GB2005/003196 is an efficient sensor, its performance can be improved by the appropriate selection of the material used to provide the coating extending between the electrodes. In particular, is has been found that an improved performance and response of the sensor may be obtained by having a layer of ion exchange material extending between the electrodes, such that when the sensor is exposed to a gas stream containing water vapour an electrical contact is established by the ion exchange material between the electrodes. Such a sensor can provide an improved indication of the lung function of a patient or subject and assist in the ready examination of a patient and diagnosis of abnormalities in the operation and performance of the lungs and respiratory system.

According to the present invention there is provided a sensor for sensing a target substance in a gas stream, the sensor comprising:

   a sensing element disposed to be exposed to the gas stream, the sensing element comprising:

      a working electrode;
      a counter electrode; and
      a layer of ion exchange material extending between the working electrode and the counter electrode; whereby contact of the ion exchange layer with the gas stream forms an electrical contact between the working and counter electrodes..

In the present specification, references to an ion exchange material are to a material having ion exchange properties, such that contact with the components of a gas stream results in a change in the conductivity of the layer between the electrodes. The ion exchange material acts as the support medium for electrical conduction to occur. In particular, in the presence of water in the ion exchange material, it allows a hydrated ionic layer to form between the electrodes. The layer of ion exchange material provides a medium that is highly controllable and hydrates uniformly to provide a suitable medium for conduction to occur.

Suitable ion exchange materials for use in the sensor of the present invention are those having a high proton conductivity, good chemical stability, and the ability to retain sufficient mechanical integrity. The ion exchange material should have a high affinity for the species present in the gas stream being analysed, in particular for the various components that are present in the exhaled breath of a subject or patient.

Suitable ion exchange materials are known in the art and are commercially available products.

Particularly preferred ion exchange material are the ionomers, a class of synthetic polymers with ionic properties. A particularly preferred group of ionomers are the sulphonated tetrafluoroethylene copolymers. An especially preferred ionomer from this class is Nafion ®, available commercially from Du Pont. The sulphonated tetrafluoroethylene copolymers have superior conductive properties due to their proton conducting capabilities. The sulphonated tetrafluoroethylene copolymers can be manufactured with various cationic conductivities. They also exhibit excellent thermal and mechanical stability and are biocompatible, thus making them suitable materials for use in the controlled electrode coating.

Other suitable ion exchange materials include polyether ether ketones (PEEK), poly(arylene-ether-sulfones) (PSU), PVDF-graft styrenes, acid doped polybenimidazoles (PBI) and polyphosphazenes.

The ion exchange material may be present in the sensor in the dry state, in which case the material will require the addition of water, for example as water vapour present in the gas stream. This is the case when the sensor is used to analyse the exhaled breath of a human or animal, where water vapour in varying amounts is present. Alternatively, the ion exchange material may be present with water in a saturated or partially-saturated state, in which case a dry gas stream may be analysed. In such a case, the output of the sensor will change in response to a change in the conductance of the ion exchange material, due to the dissolution of ions in the water present.

The thickness of the ion exchange material will determine the response of the sensor to changes in the composition of the gas stream in contact with the ion exchange layer. To minimize internal resistance within the sensor, it is preferred to use an ultra thin ion exchange layer.

The ion exchange layer may comprise a single ion exchange material or a mixture of two or more such materials, depending upon the particular application of the sensor.

The ion exchange layer may consist of the ion exchange material in the case the material exhibits the required level of chemical and mechanical stability and integrity for the working life of the sensor. Alternatively, the ion exchange layer may comprise an inert support for the ion exchange material. Suitable supports include oxides, in particular metal oxides, including aluminium oxide, titanium oxide, zirconium oxides and mixtures thereof. Other suitable supports include oxides of silicon and the various natural and synthetic clays.

In one particularly preferred embodiment, the ion exchange layer comprises, in addition to the ion exchange material and inert filler, if present, a mesoporous material. In the present specification, references to a mesoporous material are to a material having pores in the range of from 1 to 75 nm, more particularly in the range of from 2 to 50 nm. The mesoporous material provides a medium that is highly controllable and hydrates uniformly to provide a suitable medium for conduction to occur.

Suitable mesoporous materials for use in the sensor of the present invention are known in the art and commercially available, and include Zeolites. Zeolites are a particularly preferred component for inclusion in the ion exchange layer in the sensor of the present invention. One preferred zeolite is Zeolite 13X. Alternative mesoporous materials for use are Zeolite 4A or Zeolite P. The ion exchange layer may contain one or a combination of zeolite materials.

The granularity and thickness of the mesoporous material will determine the response of the sensor to changes in the composition of the gas stream in contact with the ion exchange layer. To minimize internal resistance within the sensor, it is preferred to use an ultra thin layer containing mesoporous material.

The mesoporous material is preferably dispersed in the ion exchange layer, most preferably as a fine dispersion. The mesoporous material is preferably dispersed as particles having a particle size in the range of from 0.5 to 20 $\mu$m, more preferably from 1 to 10 $\mu$m. In one embodiment, the mesoporous material is applied to the electrodes as a suspension of particles in a suitable solvent, with the solvent being allowed to evaporate to leave a fine dispersion of particles over the electrodes. Ion exchange material is then applied over the mesoporous dispersion. The mesoporous material is preferably applied in a concentration of from 0.01 to 1.0 g, as a uniform suspension in 10 ml of solvent, into which the electrode assembly is dipped one or more times. More preferably, the mesporous material is applied in a concentration of from 0.05 to 0.5 g per 10 ml of solvent, especially about 0.1g per 10 ml of solvent. Suitable solvents for use in the application of the mesoporous material are known in the art and include alcohols, in particular methanol, ethanol and higher aliphatic alcohols. Other suitable techniques for applying the mesoporous material include dry aerosol deposition, spray pyrolysis, screen printing, in-situ crystal growth, hydrothermal growth, sputtering, and autoclaving,

It has been found that the sparse population of mesoporous particles within the (continuous) ion exchange film affords the highest discrimination towards the detection of target species in the gas stream, in particular water vapour. Examination under a scanning electron microscope (SEM) of a preferred arrangement reveals a density of mesoporous particles such that each particle is, on average, distanced several body diameters, in particular from 1 to 5 body diameters, more preferably from 1 to 3 body diameters, away from the nearest neighbour.

It has also been found that thick films of ion exchange material degrade the performance of the sensor, as do thick continuous coats of the mesoporous material. In other words, it is the combination of a thin ion exchange layer and sparse population of mesoporous particles that performs best.

The sensor is particularly suitable for the detection of carbon dioxide, in particular carbon dioxide present in the exhaled breath of a person or animal. The sensor is also particularly suitable for the detection of water vapour in a gas stream. In the case of an exhaled gas stream, the measurement of the water vapour concentration exhaled by the subject allows an accurate determination of the carbon dioxide content of the exhaled breath to be determined. This feature renders the sensor particularly advantageous in the analysis of gas streams exhaled by humans and animals. In addition, the sensor provides a fast and accurate response to changes in the composition of the gas stream being analysed. These features make the sensor of the present invention particularly suitable for use as a capnographic sensor in the analysis of exhaled breath of a subject.

The present invention provides a sensor that is particularly compact and of very simple construction. In addition, the sensor may be used at ambient temperature conditions, without the need for any heating or cooling, while at the same time producing an accurate measurement of the target substance concentration in the gas being analysed.

The sensor preferably comprises a housing or other protective body to enclose and protect the electrodes. The sensor may comprise a passage or conduit to direct the stream of gas directly onto the electrodes. In a very simple arrangement, the sensor comprises a conduit or tube into which the two electrodes extend, so as to be contacted directly by the gaseous stream passing through the conduit or tube. When the sensor is intended for use in the analysis of the breath of a patient, the conduit may comprise a mouthpiece, into which the patient may exhale. Alternatively, the sensor may be formed to have the electrodes in an exposed position on or in the housing, for direct measurement of a bulk gas stream. The precise form of the housing, passage or conduit is not critical to the operation or performance of the sensor and may take any desired form. It is preferred that the body or housing of the sensor is prepared from a non-conductive material, such as a suitable plastic.

As noted above, in one embodiment, the sensor relies upon the presence of water vapour in the gaseous stream being analysed to hydrate the ion exchange layer. If insufficient water vapour is present in the gaseous stream, the sensor may be provided with a means for increasing the water vapour content of the gas stream. Such means may include a reservoir of water and a dispenser, such as a spray, nebuliser or aerosol.

The electrodes may have any suitable shape and configuration. Suitable forms of electrode include points, lines, rings and flat planar surfaces. The effectiveness of the sensor can depend upon the particular arrangement of the electrodes and may be enhanced in certain embodiments by having a very small path length between the adjacent electrodes. This may be achieved, for example, by having each of the working and counter electrodes comprise a plurality of electrode portions arranged in an alternating, interlocking pattern, that is in the form of an array of interdigitated electrode portions, in particular arranged in a concentric pattern.

The electrodes are preferably oriented as close as possible to each other, to within the resolution of the manufacturing technology. The working and counter electrode can be between 10 to 1000 microns in width, preferably from 50 to 500 microns. The gap between the working and counter electrodes can be between 20 and 1000 microns, more preferably from 50 to 500 microns. The optimum track-gap distances are found by routine experiment for the particular electrode material, geometry, configuration, and substrate under consideration. In a preferred embodiment the optimum working electrode track widths are from 50 to 250 microns, preferably about 100 microns, and the counter electrode track widths are from 50 to 750 microns, preferably about 500 microns. The gaps between the working and counter electrodes are preferably about 100 microns.

The counter electrode and working electrode may be of equal size. However, in one preferred embodiment, the surface area of the counter electrode is greater than that of the working electrode to avoid restriction of the current transfer. Preferably, the counter electrode has a surface area at least twice that of the working electrode. Higher ratios of the surface area of the counter electrode and working electrode, such as at least 3:1, preferably at least 5:1 and up to 10:1 may also be employed. The thickness of the electrodes is determined by the manufacturing technology, but has no direct influence on the electrochemistry. The magnitude of the resultant electrochemical signal is determined principally by exposed surface area, that is the surface area of the electrodes directly exposed to and in contact with the gaseous stream. Generally, an increase in the surface area of the electrodes will result in a higher signal, but may also result in increased susceptibility to noise and electrical interference. However, the signals from smaller electrodes may be more difficult to detect.

The electrodes may be supported on a substrate. Suitable materials for the support substrate are any inert, non-conducting material, for example ceramic, plastic, or glass. The substrate provides support for the electrodes and serves to keep them in their proper orientation. Accordingly, the substrate may be any suitable supporting medium. It is important that the substrate is non-conducting, that is electrically insulating or of a sufficiently high dielectric coefficient.

The electrodes may be disposed on the surface of the substrate, with the layer of ion exchange material extending over the electrodes and substrate surface. Alternatively, the ion exchange material may be applied directly to the substrate, with the electrodes being disposed on the surface of the ion exchange layer. This would have the advantage of providing mechanical strength and a thin layer of base giving greater control of path length.

The ion exchange material is conveniently applied to the surface of the substrate by evaporation from a suspension or solution in a suitable solvent. For example, in the case of sulphonated tetrafluoroethylene copolymers, a suitable solvent is methanol. The suspension or solution of the ion exchange material may also comprise the inert support or a precursor thereof, if one is to be present in the ion exchange layer.

To improve the electrical insulation of the electrodes, the portions of the electrodes that are not disposed to be in contact with the gaseous stream (that is the non-operational portions of the electrodes) may be coated with a dielectric material, patterned in such a way as to leave exposed the active portions of the electrodes.

While the sensor operates well with two electrodes, as hereinbefore described, arrangements with more than two electrodes, for example including a third or reference electrode, as is well known in the art. The use of a reference electrode provides for better potentiostatic control of the applied voltage, or the galvanostatic control of current, when the "iR drop" between the counter and working electrodes is substantial. Dual 2-electrode and 3-electrode cells may also be employed.

A further electrode, disposed between the counter and working electrodes, may also be employed. The temperature of the gas stream may be calculated by measuring the end-to-end resistance of the electrode. Such techniques are known in the art.

The electrodes may comprise any suitable metal or alloy of metals, with the proviso that the electrode does not react with the electrolyte or any of the substances present in the gas stream. Preference is given to metals in Group VIII of the Periodic Table of the Elements (as provided in the Handbook of Chemistry and Physics, 62nd edition, 1981 to 1982, Chemical Rubber Company). Preferred Group VIII metals are rhenium, palladium and platinum. Other suitable metals include silver and gold. Preferably, each electrode is prepared from gold or platinum. Carbon or carbon-containing materials may also be used to form the electrodes.

The electrodes of the sensor of the present invention may be formed by printing the electrode material in the form of a thick film screen printing ink onto the substrate. The ink consists of four components, namely the functional component, a binder, a vehicle and one or more modifiers. In the case of the present invention, the functional component forms the conductive component of the electrode and comprises a powder of one or more of the aforementioned metals used to form the electrode.

The binder holds the ink to the substrate and merges with the substrate during high temperature firing. The vehicle acts as the carrier for the powders and comprises both volatile components, such as solvents and non-volatile components, such as polymers. These materials evaporate during the early stages of drying and firing respectively. The modifiers comprise small amounts of additives, which are active in controlling the behaviour of the inks before and after processing.

Screen printing requires the ink viscosity to be controlled within limits determined by rheological properties, such as the amount of vehicle components and powders in the ink, as well as aspects of the environment, such as ambient temperature.

The printing screen may be prepared by stretching stainless steel wire mesh cloth across the screen frame, while maintaining high tension. An emulsion is then spread over the entire mesh, filling all open areas of the mesh. A common practice is to add an excess of the emulsion to the mesh. The area to be screen printed is then patterned on the screen using the desired electrode design template.

The squeegee is used to spread the ink over the screen. The shearing action of the squeegee results in a reduction in the viscosity of the ink, allowing the ink to pass through the patterned areas onto the substrate. The screen peels away as the squeegee passes. The ink viscosity recovers to its original state and results in a well defined print. The screen mesh is critical when determining the desired thick film print thickness, and hence the thickness of the completed electrodes.

The mechanical limit to downward travel of the squeegee (downstop) should be set to allow the limit of print stroke to be 75 - 125um below the substrate surface. This will allow a consistent print thickness to be achieved across the substrate whilst simultaneously protecting the screen mesh from distortion and possible plastic deformation due to excessive pressure.

To determine the print thickness the following equation can be used:

$$Tw = (Tm \times Ao) + Te$$

Where Tw = Wet thickness (um);
Tm = mesh weave thickness (um);
Ao = % open area;
Te = Emulsion thickness (um).

After the printing process the sensor element needs to be levelled before firing. The levelling permits mesh marks to fill and some of the more volatile solvents to evaporate slowly at room temperature. If all of the solvent is not removed in this drying process, the remaining amount may cause problems in the firing process by polluting the atmosphere surrounding the sensor element. Most of the solvents used in thick film technology can be completely removed in an oven at 150 °C when held there for 10 minutes.

Firing is typically accomplished in a belt furnace. Firing temperatures vary according to the ink chemistry. Most commercially available systems fire at 850 °C peak for 10 minutes. Total furnace time is 30 to 45 minutes, including the time taken to heat the furnace and cool to room temperature. Purity of the firing atmosphere is critical to successful processing. The air should be clean of particulates, hydrocarbons, halogen-containing vapours and water vapour.

Alternative techniques for preparing the electrodes and applying them to the substrate, if present, include spin/sputter coating and visible/ultraviolet/laser photolithography. In order to avoid impurities being present in the electrodes, which may alter the electrochemical performance of the sensor, the electrodes may be prepared by electrochemical plating. In particular, each electrode may be comprised of a plurality of layers applied by different techniques, with the lower layers be prepared using one of the aforementioned techniques, such as printing, and the uppermost or outer layer or layers being applied by electrochemical plating using a pure electrode material, such as a pure metal.

In use, the sensor is able to operate over a wide range of temperatures. However, the need for water vapour to be present in the gaseous stream be analysed requires the sensor to be at a temperature above the freezing point of water and above the dew point. The sensor may be provided with a heating means in order to raise the temperature of the gas stream, if required.

In a further aspect, the present invention provides a method of sensing a target substance in a gas stream comprising water vapour, the method comprising:

causing the gas stream to impinge on a layer of ion exchange material extending between a working electrode and a counter electrode;
applying an electric potential across the working electrode and counter electrode;
measuring the current flowing between the working electrode and counter electrode as a result of the applied potential; and

determining from the measured current flow an indication of the concentration of the target substance in the gas stream.

The target substance in the gas stream may be a component, such as an acidic component, present in addition to water vapour. Alternatively, the target substance may be water vapour itself, in which case the sensor is used to determine the moisture content or humidity of the gas stream.

As noted above, the method of the present invention is particularly suitable for use in the detection of carbon dioxide in a gas stream, in particular in the exhaled breath of a human or animal subject.

During operation, the impedance between the counter and working electrodes indicates the relative humidity and, if being measured, the target substance content of the gaseous stream, which may be electronically measured by a variety of techniques.

The method of the present invention may be carried out using a sensor as hereinbefore described.

Should the gas stream contain too little water vapour for operation, additional water may be added to the gas before contact with the electrodes takes place.

The method requires that an electric potential is applied across the electrodes. In one simple configuration, a voltage is applied to the counter electrode, while the working electrode is connected to earth (grounded). In its simplest form, the method applies a single, constant potential difference across the working and counter electrodes. Alternatively, the potential difference may be varied against time, for example being pulsed or swept between a series of potentials. In one embodiment, the electric potential is pulsed between a so-called 'rest' potential, at which no reaction occurs, and a reaction potential.

In operation, a linear potential scan, multiple voltage steps or one discrete potential pulse are applied to the working electrode, and the resultant Faradaic reduction current is monitored as a direct function of the dissolution of target molecules in the water bridging the electrodes.

The measured current in the sensor element is usually small. The current is converted to a voltage using a resistor, R. As a result of the small current flow, careful attention to electronic design and detail may be necessary. In particular, special "guarding" techniques may be employed. Ground loops need to be avoided in the system. This can be achieved using techniques known in the art.

The current that passes between the counter and working electrodes is converted to a voltage and recorded as a function of the carbon dioxide concentration in the gaseous stream. The sensor responds faster by pulsing the potential between two voltages, a technique known in the art as 'Square Wave Voltammetry'. Measuring the response several times during a pulse may be used to assess the impedance of the sensor.

The shape of the transient response can be simply related to the electrical characteristics (impedance) of the sensor in terms of simple electronic resistance and capacitance elements. By careful analysis of the shape, the individual contributions of resistance and capacitance may be calculated. Such mathematical techniques are well known in the art. Capacitance is an unwanted noisy component resulting from electronic artifacts, such as charging, etc. The capacitive signal can be reduced by selection of the design and layout of the electrodes in the sensor. Increasing the surface area of the electrodes and increasing the distance between the electrodes are two major parameters that affect the resultant capacitance. The desired Faradaic signal resulting from the passage of current due to reaction between the electrodes may be optimized, by experiment. Measurement of the response at increasing periods within the pulse is one technique that can preferentially select between the capacitive and Faradaic components, for instance. Such practical techniques are well known in the art.

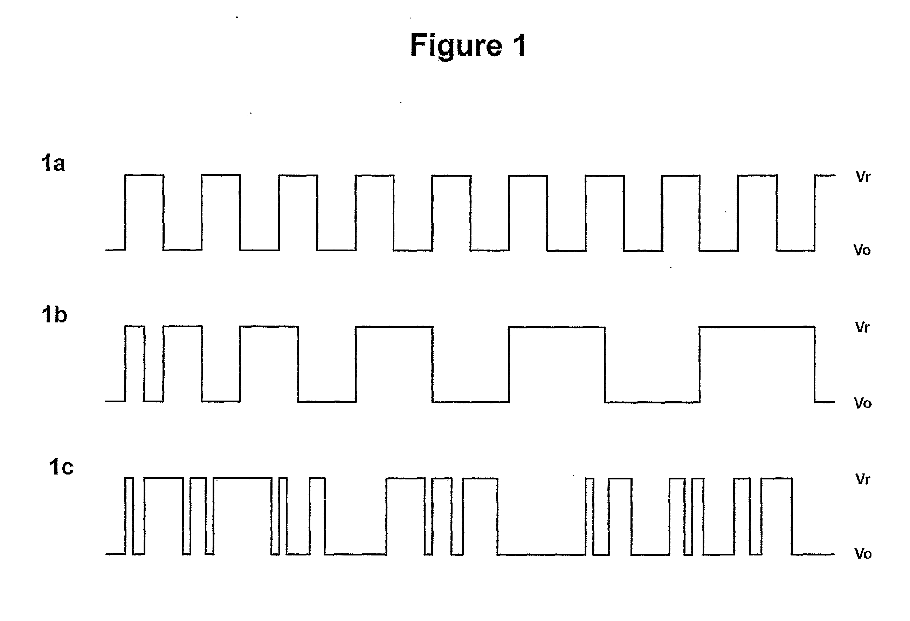

The potential difference applied to the electrodes of the sensor element may be alternately or be periodically pulsed between a rest potential and a reaction potential, as noted above. Figure 1 shows examples of voltage waveforms that may be applied. Figure 1a is a representation of a pulsed voltage signal, alternating between a rest potential, $V_0$, and a reaction potential $V_R$. The voltage may be pulsed at a range of frequencies, typically from sub-Hertz frequencies, that is from 0.1 Hz, up to 10kHz. A preferred pulse frequency is in the range of from 1 to 500 Hz. Alternatively, the potential waveform applied to the counter electrode may consist of a "swept" series of frequencies, represented in Figure 1b. A further alternative waveform shown in Figure 1c is a so-called "white noise" set of frequencies. The complex frequency response obtained from such a waveform will have to be deconvoluted after signal acquisition using techniques such as Fourier Transform analysis. Again, such techniques are known in the art.

One preferred voltage regime is 0V ("rest" potential), 250mV ("reaction" potential), and 20Hz pulse frequency.

It is an advantage of the present invention that the electrochemical reaction potential is approximately +0.2 volts, which avoids many if not all of the possible competing reactions that would interfere with the measurements, such as the reduction of metal ions and the dissolution of oxygen.

The method of the present invention is particularly suitable for use in the analysis of the exhaled breath of a person or animal. From the results of this analysis, an indication of the respiratory condition of the patient may be obtained.

Accordingly, in a further aspect, the present invention provides a method of measuring the concentration of a target substance in the exhaled breath of a subject, such as a human or animal, the method comprising:

causing the exhaled breath to impinge on a layer of ion exchange material extending between a working electrode and a counter electrode;

applying an electric potential across the working electrode and counter electrode;

measuring the current flowing between the working electrode and counter electrode as a result of the applied potential; and

determining from the measured current flow an indication of the concentration of a target substance in the exhaled breath stream.

The gas exhaled by a person or animal is often saturated in water vapour, as a result of the action of the gas exchange mechanisms taking place in the lungs of the subject. The sensor may be used to measure and monitor the water-content of the exhaled breath of a subject human or animal.

The sensor and method of the present invention are of use in monitoring and determining the lung function of a patient or subject. The method and sensor are particularly suitable for analyzing tidal concentrations of substances, such as carbon dioxide, in the exhaled breath of a person or animal, to diagnose or monitor a variety of respiratory conditions. The sensor is particularly useful for applications requiring fast response times, for example personal respiratory monitoring of tidal breathing (capnography). Capnographic measurements can be applied generally in the field of respiratory medicine, airway diseases, both restrictive and obstructive, airway tract disease management, and airway inflammation. The present invention finds particular application in the field of capnography and asthma diagnosis, monitoring and management, where the shape of the capnogram changes as a function of the extent of the disease. In particular, due to the high rate of response that may be achieved using the sensor and method of the present invention, the results may be used to provide an early alert to the onset of an asthma attack in an asthmatic patient.

Measuring the percentage saturation and variation of water vapour in the exhaled breath of a subject or animal may also be used in the diagnosis of Adult Respiratory Distress Syndrome (ARDS), an end-stage life-threatening lung disease. ARDS is characterized by pulmonary intersititial oedema. In a subject in good health, there is normally a steady state distribution of water between blood and tissues in the lung. The outward filtration of water (due to positive transcapillary hydrostatic pressure) is balanced by re-absorption from the insterstitium (by lymphatic drainage). ARDS upsets this balance. There are a number of phases to the disease, but increased capillary permeability commonly causes accumulation of water in the lungs. Therefore, monitoring the amount and variation in the water exhaled by a patient may be useful in the diagnosis and management of ARDS.

Embodiments of the present invention will now be described, by way of example only, having reference to the accompanying drawings, in which:

Figures 1a, 1b and 1c are voltage versus time representations of possible voltage waveforms that may be applied to the electrodes in the method of the present invention, as discussed hereinbefore;

Figure 2 is a cross-sectional representation of one embodiment of the sensor of the present invention;

Figure 3 is an isometric schematic view of a face of one embodiment of the sensor element according to the present invention;

Figure 4 is an isometric schematic view of an alternative embodiment of the sensor element of the sensor of the present invention;

Figure 5 is a schematic view of a potentiostat electronic circuit that may be used to excite the electrodes of the sensor element;

Figure 6 is a schematic view of a galvanostat electronic circuit that may be used to excite the electrodes;

Figure 7 is a schematic representation of a breathing tube adaptor for use in the sensor of the present invention;

Figure 8 is a flow-diagram providing an overview of the inter-connection of sensor elements and their connection into a suitable measuring instrument of an embodiment of the present invention;

Figure 9 is a SEM photograph of particles of zeolite dispersed across the electrodes of a sensor of the present invention;

Figure 10 is a capnogram showing the variation in the concentration of water vapour with time obtained from the analysis of the inhaled and exhaled gas streams of a patient using a sensor of the present invention; and

Figure 11 is a diagrammatic representation of one form of voltage signal applied to the sensor of the present invention and the measured current response.

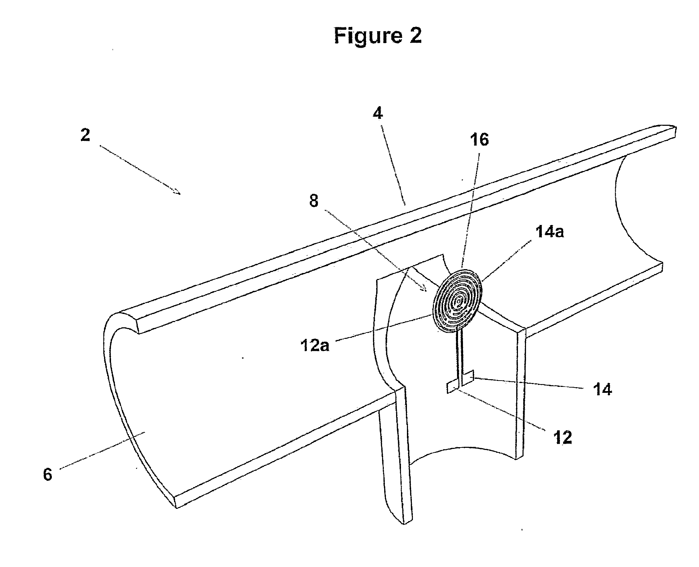

Referring to Figure 2, there is shown a sensor according to the present invention. The sensor is for analyzing the carbon dioxide content and humidity of exhaled breath. The sensor, generally indicated as 2, comprises a conduit 4, through which a stream of exhaled breath may be passed. The conduit 4 comprises a mouthpiece 6, into which the patient may breathe.

A sensing element, generally indicated as 8, is located within the conduit 4, such that a stream of gas passing through the conduit from the mouthpiece 6 is caused to impinge upon the sensing element 8. The sensing element 8 comprises a support substrate 10 of an inert material, onto which is mounted a working electrode 12 and a reference electrode 14. The working electrode 12 and reference electrode 14 each comprise a plurality of electrode portions, 12a and 14a, arranged in concentric circles, so as to provide an interwoven pattern minimizing the distance between adjacent portions of the working electrode 12 and reference electrode 14. In this way, the current path between the two electrodes is kept to a minimum.

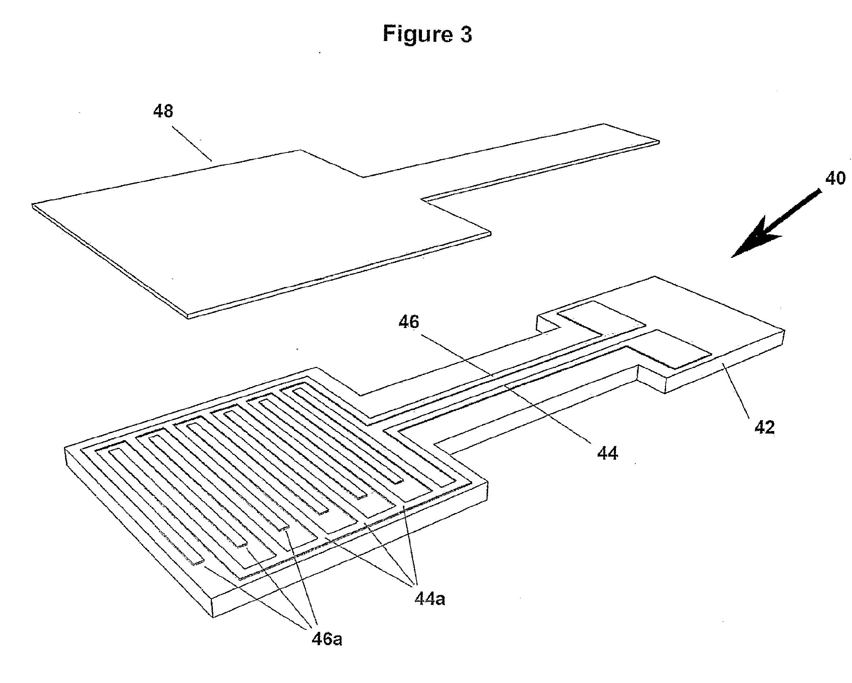

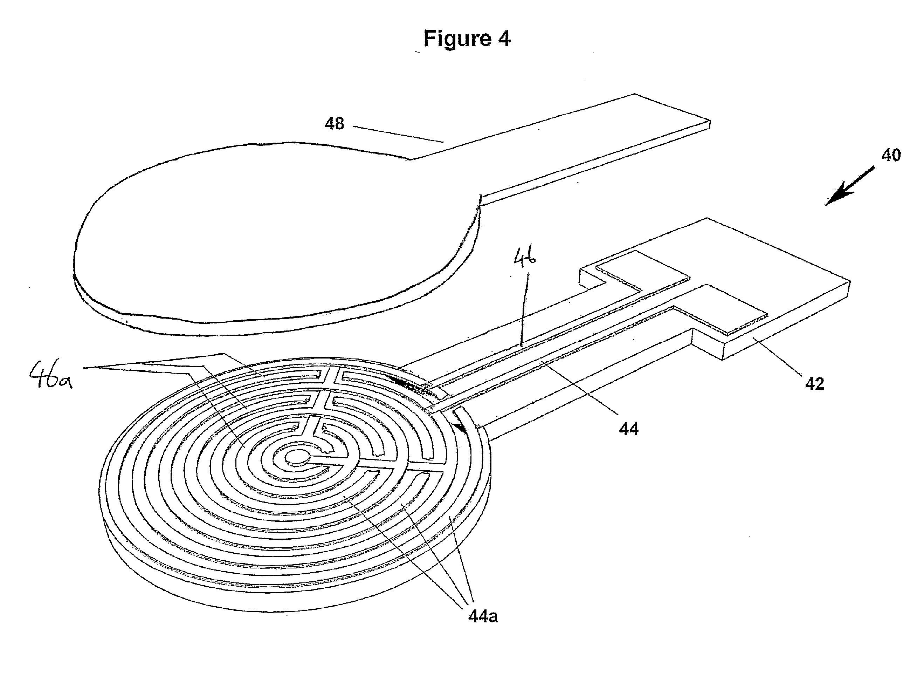

A layer 16 of insulating or dielectric material extends over a portion of both the working and counter electrodes 12 and 14, leaving the portions 12a and 14a of each electrode exposed to be in direct contact with a stream of gas passing through the conduit 4. The arrangement of the support, electrodes 12 and 14, and the solid electrolyte precursor is shown in more detail in Figures 3 and 4.

Referring to Figure 3, there is shown an exploded view of a sensor element, generally indicated as 40, comprising a substrate layer 42. A working electrode 44 is mounted on the substrate layer 42 from which extend a series of elongated electrode portions 44a. Similarly, a reference electrode 46 is mounted on the substrate layer 42 from which extends a series of electrode portions 46a. As will be seen in Figure 3, the working electrode portions 44a and the reference electrode portions 46a extend one between the other in an intimate, interdigitated array, providing a large surface area of exposed electrode with minimum separation between adjacent portions of the working and reference electrodes. A layer of ion exchange material 48 overlies the working and reference electrodes 44, 46.

The ion exchange material consists of Nafion ®, a commercially available sulphonated tetrafluoroethylene copolymer.

The ion exchange material 48 is applied by the repeated immersion in a suspension or slurry of the Nafion ® in a suitable solvent, in particular methanol. The pH will determine the ion exchanger characteristics of the Nafion ®. It is possible to manufacture a Nafion ® coating with principally $H^+$, $K^+$, $Na^+$ and $Ca^{2+}$ as the cationic exchanger. The sensor element is dried to evaporate the solvent after each immersion and before the subsequent immersion. Other materials may be incorporated into the ion exchange layer by subsequent immersion in additional solutions or suspensions. The number of immersions is determined by the required thickness of the ion exchange layer, and the chemical composition is determined by the number and variety of additional solutions that the sensor is dipped into.

It will be obvious that there are a number of other means whereby the thickness and composition of the coating may be similarly achieved, such as: pad, spray, screen and other mechanical methods of printing. Such techniques are well known in the field.

An alternative electrode arrangement is shown in Figure 4, in which components common to the sensor element of Figure 3 are identified with the same reference numerals. It will be noted that the working electrode portions 44a and the reference electrode portions 46a are arranged in an intimate circular array. The electrodes and substrate are coated in a layer of ion exchange material, as described above in relation to Figure 3.

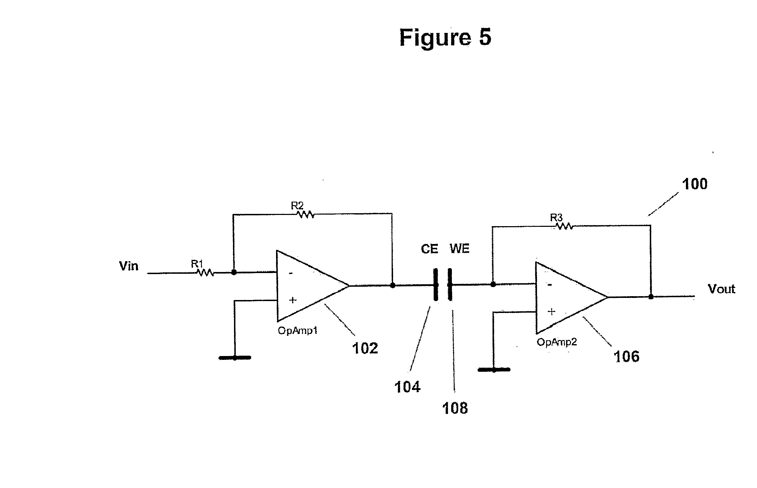

Referring to Figure 5, there is shown a potentiostat electronic circuit that may be employed to provide the voltage applied across the working and reference electrodes of the sensor of the present invention. The circuit, generally indicated as 100, comprises an amplifier 102, identified as 'OpAmp1', acting as a control amplifier to accept an externally applied voltage signal $V_{in}$. The output from OpAmp1 is applied to the control (counter) electrode 104. A second amplifier 106, identified as 'OpAmp2' converts the passage of current from the counter electrode 104 to the working electrode 108 into a measurable voltage ($V_{out}$). Resistors R1, R2 and R3 are selected according to the input voltage, and measured current.

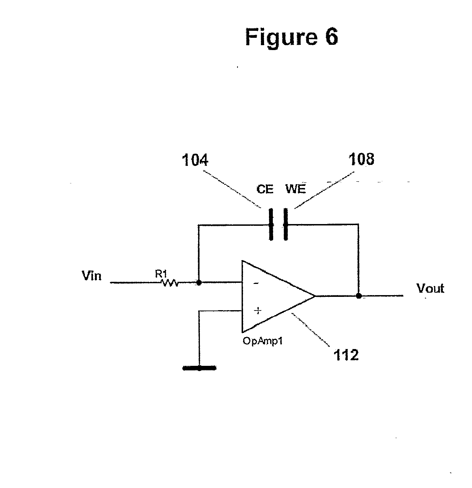

An alternative galvanostat circuit for exciting the electrodes of the sensor is shown in Figure 6. The control and working electrodes 104 and 108 are connected between the input and output of a single amplifier 112, indicated as 'OpAmp1'. Again, resistor R1 is selected according to the desired current.

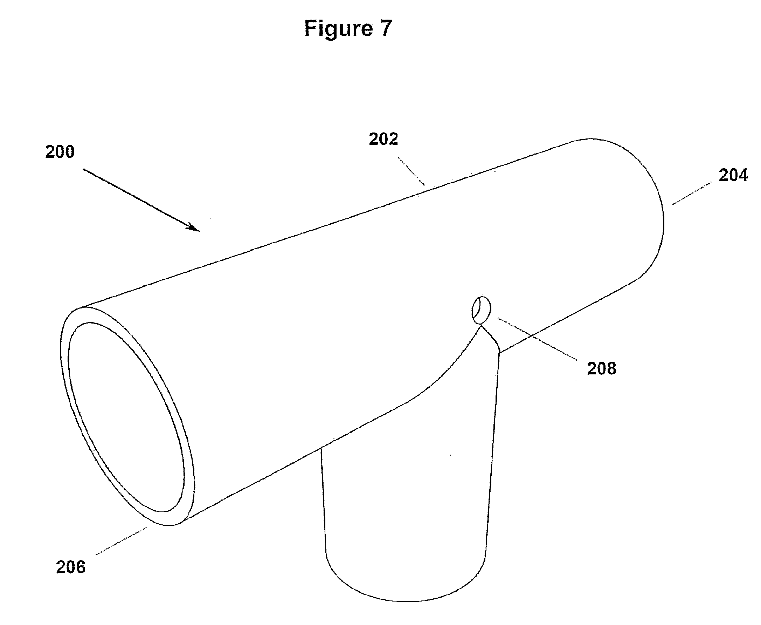

Turning to Figure 7, an adaptor for monitoring the breath of a patient is shown. A sensor element is mounted within the adaptor and oriented directly into the air stream flowing through the adaptor, in a similar manner to that shown in Figure 2 and described hereinbefore. The preferred embodiment illustrated in Figure 7 comprises and adaptor, generally indicated as 200, having a cylindrical housing 202 having a male-shaped (push-fit) cone coupling 204 at one end and a female-shaped (push-fit) cone coupling 206 at the other. A side inlet 208 is provided in the form of an orifice in the cylindrical housing 202, allowing for the adaptor to be used in the monitoring of the tidal breathing of a patient, as described in more detail in Example 2 below. The side inlet 208 directs gas onto the sensor element during inhalation by a patient through the device. The monitoring of tidal breathing may be improved by the provision of a one-way valve on the outlet of the housing 202.

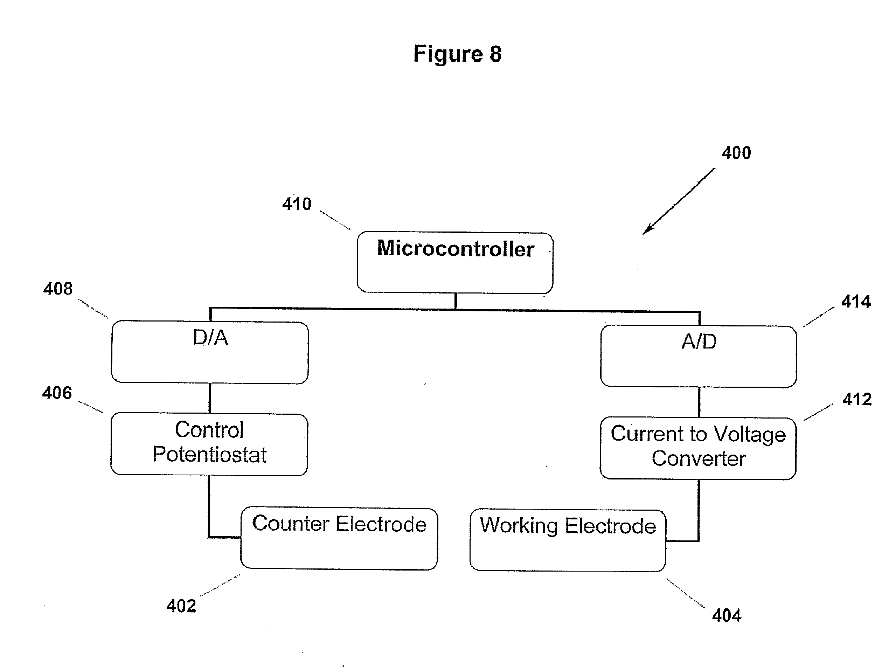

With reference to Figure 8 there is shown in schematic form the general layout of a sensor system according to the present invention. The system, generally indicated as 400, comprises a sensor element having a counter electrode 402 and a working electrode 404. The counter electrode 402 is supplied with a voltage by a control potentiostat 406, for example of the form shown in Figure 5 and described hereinbefore. The input signal for the control potentiostat 406 is provided by a digital-to-analog converter (D/A) 408, itself being provided with a digital input signal from a microcontroller 410. The output signal generated by the sensing element is in the form of a current at the working electrode 404, which is fed to a current-to-voltage converter 412, the output of which is in turn fed to an analog-to-digital converter (A/D) 414. The microcontroller 410 receives the output of the A/D converter 414, which it employs to generate a display indicating the concentration of the target substance in the gas stream being monitored. The display (not shown in Figure 8 for reasons of clarity) may be any suitable form of display, for example an audio display or visual display. In one preferred embodiment, the microcontroller 410 generates a continuous display of the concentration of the target substance, this arrangement being particularly useful in the monitoring of the tidal breathing of a patient.

The sensors of the present invention may be employed individually, or as a series of sensor elements connected sequentially together in-line to measure a series of gases from a single gas stream. For example, a series of sensors may be employed to analyse the exhaled breath of a patient. In addition, two or more sensors may be used to compare the composition of the inhaled and exhaled breath of a patient.

The present invention will be further illustrated by way of the following example.

EXAMPLE

A sensor having the general configuration shown in Figures 2 and 3 was prepared. The electrodes were coated with an ion exchange layer comprising a commercially available sulphonated tetrafluoroethylene copolymer (Nafion ®, ex Du Pont) and zeolite 4A. The coating was prepared as follows:

A suspension of the zeolite material was suspended in 10ml of methanol. The zeolite had a uniform range of particle sizes, about 1 micron particle diameter.

The suspension was sonicated for 10 minutes, to ensure even dispersion of the Zeolite within the solution. An ultrasonic bath or probe may also be used. The electrode to be coated was then immersed into the solution and held for 2 seconds before withdrawal. The electrode was laid flat and the solvent allowed to naturally evapourate. Forced air convection may also be used to accelerate the evaporation of the solvent, if necessary.

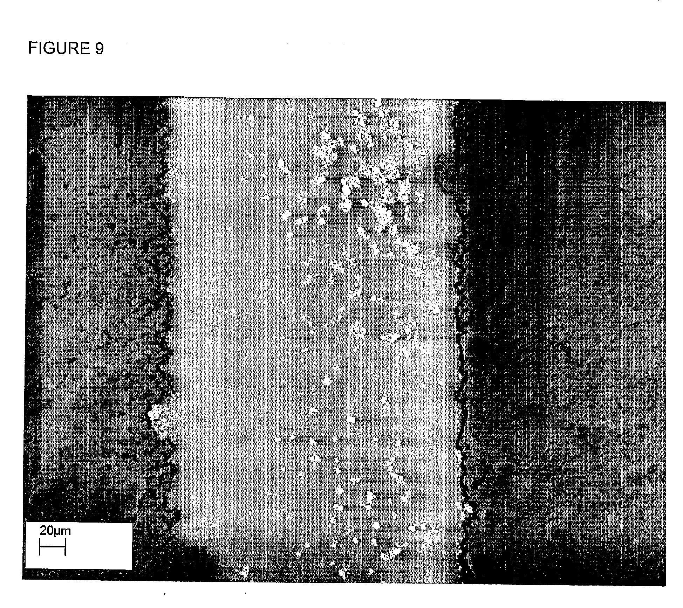

The electrodes were inspected using SEM to determine the distribution of zeolite particles across the electrodes. The results are shown in Figure 9. As can be seen, the zeolite particles are finely dispersed across the surface of the electrode, with the spacing between particles generally being at least one particle diameter.

With the sensor still in the horizontal position, a minute volume of Nafion polymer was then dispensed onto the surface of the sensor using a syringe, and spread across the entire surface of the sensor using the edge of the syringe needle used to dispense the fluid. The solvent was again left to naturally evaporate away. The volume was such to ensure complete coverage of the surface area of the sensor, and to ensure that the resultant thickness of the film was as small as possible. Typical volumes range from 1 to 10ul to cover an area of 1cm$^2$, preferably 2ul. The resultant thickness of the residual layer (after evaporation of the solvent) should be reasonably thin, consistent with the intended application. Practically, layer thicknesses of 10 to 1000nm can be achieved using this method, preferably 100nm.

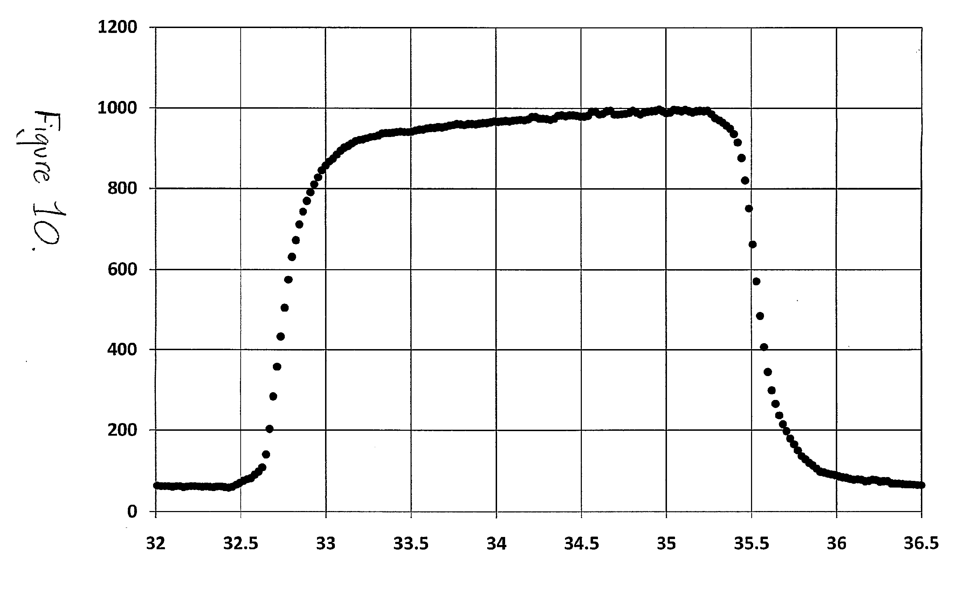

The sensor was used to analyse the composition of the breath exhaled by a patient,, in particular the water vapour content of the exhaled breath, by having the patient inhale and exhale through the assembly of Figure 1. The resulting capnogram is shown in Figure 10, from which it can be seen that the sensor produced a very accurate trace of the variation in the concentration of water in the exhaled breath over time.

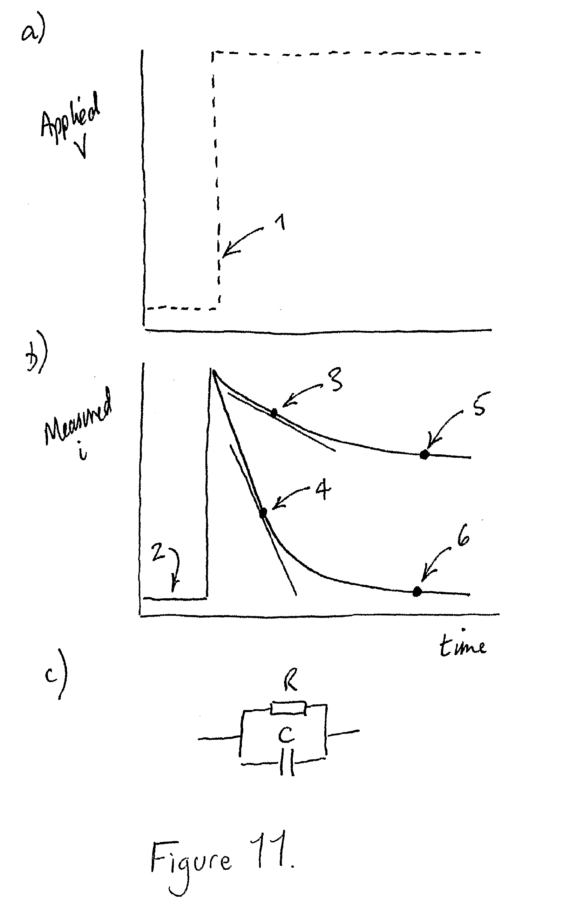

Referring to Figure 11, there is shown in Figure 11a a graphical representation of the voltage applied across the electrodes of the sensor. As shown, the step voltage (1) is applied to the counter electrode. The step change should preferably be as instant and immediate as possible. Figure 11b shows two illustrations of current transient responses received from the working electrode (after current-to-voltage conversion). The responses are both characterised by an immediate current transient (a 'spike') which decays exponentially with time. The upper curve in Figure 11b illustrates a sensor reacting to high concentrations of water vapour, and the lower curve that of the same sensor reacting to a low water vapour concentration. The measured current (after the step change) may be used to estimate concentration. For example, the value for the slope at point (3) or (4), or the absolute value for the current at point (5) or (6), may be used to estimate concentration. The reaction mechanism of the coating applied across the surface of the electrodes may be considered (in electronic equivalents) as a combination of simple resistance and capacitance as shown in Figure 11c. It is the capacitor that contributes mostly towards the 'spike' and exponential decay seen in Figure 11b, and which varies most when the sensor is exposed to water vapour.

The following section of the description consists of numbered paragraphs simply providing statements of the invention already described herein. The numbered paragraphs in this section are not claims. The claims are set forth below in the later section headed "claims".

1. A sensor for sensing a target substance in a gas stream, the sensor comprising:

a sensing element disposed to be exposed to the gas stream, the sensing element comprising:

a working electrode;
a counter electrode; and

a layer of ion exchange material extending between the working electrode and the counter electrode; whereby contact of the ion exchange layer with the gas stream forms an electrical contact between the working and counter electrodes.

2. The sensor according to 1, wherein the ion exchange material is an ionomer, especially a sulphonated tetrafluoroethylene copolymer.

3. The sensor according to any preceding wherein the ion exchange layer comprises a mesoporous material.

4. The sensor according to .3, wherein the mesoporous material is a zeolite, in particular zeolite 13, zeolite 4A or a mixture thereof.

5. The sensor according to either of 3 or 4, wherein the mesoporous material is distributed as a fine dispersion.

6. The sensor according to any preceding , wherein the ion exchange material comprises water, or condensed water vapour.

7. The sensor according to any preceding , wherein the target substance is an acidic substance, in particular carbon dioxide, or water.

8. The sensor according to any preceding , further comprising a conduit through which the gas stream is channeled to impinge upon the sensing element.

9. The sensor according to 8, wherein the conduit comprises a mouthpiece into which a patient may exhale.

10. The sensor according to any preceding wherein the working electrode and counter electrode are in a form selected from a point, a line, rings and flat planar surfaces.

11. The sensor according to any preceding, wherein one or both of the working electrode and the counter electrode comprises a plurality of electrode portions.

12. The sensor according to 11, wherein both the working electrode and the counter electrode comprise a plurality of electrode portions arranged in an interlocking pattern.

13. The sensor according to 11, wherein the electrode portions are arranged in a concentric pattern.

14. The sensor according to any preceding wherein the surface area of the counter electrode is greater than the surface area of the working electrode.

15. The sensor according to 14, wherein the ratio of the surface area of the counter electrode to the working electrode is at least 2:1, more preferably at least 5:1.

16. The sensor according to any preceding , wherein the electrodes are supported on an inert substrate.

17. The sensor according to any preceding , wherein each electrode comprises a metal selected from Group VIII of the Periodic Table of the Elements, copper, silver and gold, preferably gold or platinum.

18. The sensor according to any preceding further comprising a layer of insulating material disposed over a portion of each electrode, the insulating layer being so shaped as to leave a portion of each electrode exposed for direct contact with a gas stream.

19. The sensor according to any preceding further comprising a reference electrode.

20. The sensor according to any preceding wherein the electrodes are mounted on a substrate, the electrodes being applied to the substrate by thick film screen printing, spin/sputter coating or visible/ultraviolet/laser photolithography.

21. The sensor according to any preceding wherein one or more electrodes is comprised of a plurality of layers, the outer layer being a layer of pure metal applied by electrochemical plating.

22. The sensor according to any preceding further comprising a heater to heat the gas stream directly impinging upon the electrodes.

23. A method of sensing a target substance in a gas stream, the gas stream comprising water vapour, the method comprising:

causing the gas stream to impinge on a layer of ion exchange material extending between a working electrode and a counter electrode; applying an electric potential across the working electrode and counter electrode; measuring the current flowing between the working electrode and counter electrode as a result of the applied potential; and determining from the measured current flow an indication of the concentration of the target substance in the gas stream.

24. The method of, 23, wherein the target substance is an acidic substance, such as carbon dioxide, water vapour or a combination thereof.

25. The method of 23 or 24, wherein a constant voltage is applied across the working electrode and the counter electrode.

26. The method of 23 or 24, wherein a variable voltage is applied across the working electrode and the counter electrode.

27. The method of 26, wherein the variable voltage alternates between a rest potential and a potential above the reaction threshold potential.

28. The method of 27, wherein the voltage is pulsed at a frequency of from 0.1Hz to 20 kHz.

29. A method of measuring the concentration of a target substance in the exhaled breath of a patient, the method comprising:

causing the exhaled breath to impinge on a layer of ion exchange material extending between a working electrode and a counter electrode; applying an electric potential across the working electrode and counter electrode; measuring the current flowing between the working electrode and counter electrode as a result of the applied potential; and determining from the measured current flow an indication of the concentration of a target substance in the exhaled breath stream.

30. The method of 29, wherein the target substance is water and/or carbon dioxide.

31. The method of 29 or 30, wherein the method is applied to determine the lung function of a patient, in particlar a patient suffering from asthma, COPD or ARDS.

32. The method of any of 29 to 31, wherein the tidal breathing of a patient is monitored.

33. A system for monitoring the composition of a gas stream comprising:

a sensor according to any of 1 to 22; a microcontroller for receiving an output from the sensor; and a display; wherein the microcontroller is programmed to generate a continuous image of the concentration of a target substance in a gas stream being analysed on the display.

34. The system of 33, wherein the sensor is adapted to be exposed to the breath of a patient.

35. The system of 33 or 34, wherein the target substance is water and/or carbon dioxide.

## Claims

1. A sensor (2) for sensing water vapour in exhaled breath, the sensor comprising:

a sensing element (8, 40) disposed to be exposed to the exhaled breath, the sensing element comprising:

a working electrode (12, 44); a counter electrode (14, 46); and a layer of ion exchange material (48) extending between the working electrode and the counter electrode;

whereby contact of the ion exchange layer with water vapour in exhaled breath forms an electrical contact between the working and counter electrodes and the sensor being configured with a microcontroller to generate a display representative of the change in concentration of water vapour over time in a breath exhalation.

2. A sensor according to claim 1, wherein the ion exchange material comprises an ionomer.

3. A sensor according to claim 2 wherein the ion exchange material comprises a sulphonated tetrafluoroethylene copolymer such as Nafion®.

4. A sensor according to any one of claims 1 to 3 wherein the ion exchange material is supported by a substrate (10, 42) comprising or consisting of an alumina substrate.

5. A sensor according to any one of claims 1 to 3 wherein the electrodes are supported by a support (10, 42) selected from a ceramic support or silicon oxide support.

6. A sensor according to any one of the preceding claims for monitoring successive exhaled breaths of tidal breathing further comprising a conduit (4, 202) through which exhaled breath is channelled to impinge upon the sensing element and an orifice (208) arranged to direct air onto the sensing element during inhalation through the sensor.

7. A sensor according to any one of the preceding claims, wherein both the working electrode and the counter electrode comprise a plurality of electrode portions arranged in an interlocking pattern.

8. A sensor according to any one of the preceding claims, wherein the working electrode and counter electrode comprise electrode portions arranged in an alternating concentric pattern.

**9.** A sensor according to any one of the preceding claims, wherein each electrode comprises a metal selected from copper, silver, gold, platinum and palladium.

**10.** A sensor according to claim 9 wherein the electrodes are gold.

**11.** A sensor according to any one of the preceding claims, wherein the electrodes have been applied to a substrate by thick film screen printing.

**13.** Use of a sensor to monitor water vapour in exhaled breath, said sensor comprising a sensing element comprising
a working electrode,
a counter electrode, and
a layer of ion-exchange material extending between the working electrode and the counter electrode whereby contact of the ion-exchange layer with water vapour in exhaled breath forms an electrical contact between the working and counter electrodes and wherein a pulsed electric potential is applied across said electrodes and the output of the sensor is used to generate a display representative of the change of water vapour over time in breath exhalation;
said use not being a method of diagnosis practised on the human or animal body.

**14.** Use according to claim 13 wherein successive exhaled breaths of tidal breathing are monitored.

**15.** Use according to claim 13 or claim 14 wherein said ion-exchange material comprises a sulphonated tetrafluoroethylene copolymer such as Nafion®.

Figure 1

1a

1b

1c

Figure 2

Figure 3

Figure 4

Figure 5

Figure 6

Figure 7

EP 2 710 955 A1

## Figure 8

EP 2 710 955 A1

FIGURE 9

Figure 10.

a)

Applied
V

1

b)

Measured
i

3

5

4

2

6

time

c)

R

C

Figure 11.

EUROPEAN SEARCH REPORT

Europäisches Patentamt
European Patent Office
Office européen des brevets

Application Number

EP 13 18 8254

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TSUNEO TATARA ET AL: "AN APNEA MONITOR USING A RAPID-RESPONSE HYGROMETER", JOURNAL OF CLINICAL MONITORING, vol. 13, 1 January 1997 (1997-01-01), pages 5-9, XP055045447, * the whole document * | 1-15 | INV. A61B5/083 G01N27/49 G01N33/497 |
| Y | FONASH S J ET AL: "A Rapid-Response, High-Sensitivity Nanophase Humidity Sensor for Respiratory Monitoring", IEEE ELECTRON DEVICE LETTERS, IEEE SERVICE CENTER, NEW YORK, NY, US, vol. 25, no. 8, 1 August 2004 (2004-08-01), pages 526-528, XP011115311, ISSN: 0741-3106, DOI: 10.1109/LED.2004.832657 * the whole document * | 1-15 | |
| Y | US 5 131 990 A (KULWICKI BERNARD M [US] ET AL) 21 July 1992 (1992-07-21) * column 2, lines 6-30 * * column 4, lines 24-54; figures 1,2 * | 1-15 | |
| Y | EP 0 299 780 A (STANFORD RES INST INT [US]) 18 January 1989 (1989-01-18) * page 7, lines 25-59; figure 5 * | 1-15 | TECHNICAL FIELDS SEARCHED (IPC)  A61B G01N |
| Y | YASUDA A ET AL: "Electrochemical carbon monoxide sensor with a Nafion<(>R) film", REACTIVE & FUNCTIONAL POLYMERS, ELSEVIER SCIENCE PUBLISHERS BV, NL, vol. 41, no. 1-3, 15 July 1999 (1999-07-15), pages 235-243, XP004172678, ISSN: 1381-5148 * abstract; figure 1 * | 1-15 | |

-/--

The present search report has been drawn up for all claims

| Place of search | Date of completion of the search | Examiner |
|---|---|---|
| Munich | 14 February 2014 | Stussi, Elisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

# EUROPEAN SEARCH REPORT

Application Number

EP 13 18 8254

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | VAN DER WAL P D ET AL: "Extremely stable Nafion based carbon monoxide sensor", SENSORS AND ACTUATORS B, ELSEVIER SEQUOIA S.A., LAUSANNE, CH, vol. 35, no. 1, September 1996 (1996-09), pages 119-123, XP004049741, ISSN: 0925-4005 * the whole document * | 1-15 | |
| Y | OTAGAWA ET AL: "Planar microelectrochemical carbon monoxide sensors", SENSORS AND ACTUATORS B, ELSEVIER SEQUOIA S.A., LAUSANNE, CH, vol. 1, no. 1-6, January 1990 (1990-01), pages 319-325, XP022084294, ISSN: 0925-4005 * abstract * | 1-15 | |
| Y | MORTIMER R J ET AL: "AC impedance characteristics of solid-state planar electrochemical carbon monoxide sensors with Nafion(R) as solid polymer electrolyte", ELECTROCHIMICA ACTA, ELSEVIER SCIENCE PUBLISHERS, BARKING, GB, vol. 47, no. 20, 5 August 2002 (2002-08-05), pages 3383-3387, XP004373173, ISSN: 0013-4686 * the whole document * | 1-15 | TECHNICAL FIELDS SEARCHED (IPC) |

-/--

The present search report has been drawn up for all claims

| Place of search | Date of completion of the search | Examiner |
|---|---|---|
| Munich | 14 February 2014 | Stussi, Elisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 13 18 8254

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TRICOLI V ET AL: "Zeolite-Nafion composites as ion conducting membrane materials", ELECTROCHIMICA ACTA, ELSEVIER SCIENCE PUBLISHERS, BARKING, GB, vol. 48, no. 18, 1 August 2003 (2003-08-01), pages 2625-2633, XP004435009, ISSN: 0013-4686 * abstract * | 1-15 | |

TECHNICAL FIELDS
SEARCHED        (IPC)

The present search report has been drawn up for all claims

| Place of search | Date of completion of the search | Examiner |
|---|---|---|
| Munich | 14 February 2014 | Stussi, Elisa |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

**ANNEX TO THE EUROPEAN SEARCH REPORT
ON EUROPEAN PATENT APPLICATION NO.**                    EP 13 18 8254

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2014

| Patent document cited in search report | | Publication date | Patent family member(s) | | Publication date |
|---|---|---|---|---|---|
| US 5131990 | A | 21-07-1992 | NONE | | |
| EP 0299780 | A | 18-01-1989 | EP | 0299780 A2 | 18-01-1989 |
| | | | JP | S6488354 A | 03-04-1989 |
| | | | US | 4900405 A | 13-02-1990 |

**EP 2 710 955 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 04001407 A
- US 4772863 A
- GB 2287543 A
- GB 2316178 A
- GB 2184549 A
- EP 0293230 A
- US 6454923 B
- GB 2005003196 W

**Non-patent literature cited in the description**

- Handbook of Chemistry and Physics. Chemical Rubber Company, 1981